(19) Europäisches Patentamt
European Patent Office
Office européen des brevets

(11) EP 3 434 194 A1

(12) **EUROPEAN PATENT APPLICATION**
published in accordance with Art. 153(4) EPC

(43) Date of publication:
**30.01.2019 Bulletin 2019/05**

(51) Int Cl.:
**A61B 10/00** (2006.01)  **A61B 3/113** (2006.01)

(21) Application number: **17770423.6**

(22) Date of filing: **24.03.2017**

(86) International application number:
**PCT/JP2017/012067**

(87) International publication number:
**WO 2017/164383 (28.09.2017 Gazette 2017/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.03.2016 JP 2016059657**

(71) Applicant: **Kyoto University**
**Kyoto-shi, Kyoto 606-8501 (JP)**

(72) Inventors:
• **MIURA, Kenichiro**
**Kyoto-shi**
**Kyoto 606-8501 (JP)**
• **OHNISHI, Yusuke**
**Kyoto-shi**
**Kyoto 606-8501 (JP)**

(74) Representative: **Prinz & Partner mbB**
**Patent- und Rechtsanwälte**
**Rundfunkplatz 2**
**80335 München (DE)**

(54) **VISUAL FILTER IDENTIFICATION METHOD AND DEVICE**

(57)    The present invention is to non-invasively, objectively, and quantitatively identify a temporal filter associated with visual function by using an eye movement. According to the invention, an initial image having a constant luminance in an entirety of the initial image, a first stimulus pattern having a mean luminance that is the same as that of the initial image, and a second stimulus pattern that induces an apparent motion in conjunction with the first stimulus pattern are presented in this order. Eye movement is measured in a period of time while the second stimulus pattern is being presented. Also, the eye movement is associated with presentation time length of the first stimulus pattern and then stored. In this process, parameters of the motion energy model are optimized so that

a difference between a measurement waveform identified by the change in eye position and by the presentation time length associated with the change in eye position and the simulation result is minimized and thereby the temporal filter that is specific to the subject is calculated.

*Fig.2*

FIRST GRAY IMAGE + FIXATION TARGET
FIRST STIMULUS PATTERN + FIXATION TARGET
SECOND STIMULUS PATTERN
SECOND GRAY IMAGE

TIME (ONE TRIAL)

**Description**

FIELD OF THE INVENTION

The present invention relates to a method and apparatus for non-invasively, objectively, and quantitatively identifying a visual filter which represents a fundamental property of visual function.

BACKGROUND

Visual functions can be estimated through several techniques such as visual test and perimetry test. These techniques are used in estimating spatial resolution and visible extent of visual system. A dynamic visual acuity test is also provided for measuring motion acuity which is exerted in watching moving objects. This technique is used in estimating visual spatial resolution with respect to the moving objects.

Other techniques or methods have been proposed, such as method for measuring Critical Flicker Frequency (CFF), and method for determining perceptual contrast sensitivities for respective temporal frequencies and thereby quantifying characteristics of frequency characteristics. Additionally, another method has also been proposed for estimating temporal filter (See Non-patent literature 1).

CONVENTIONAL ART(S)

NON-PATENT LITERATURE

Burr & Morrone, J. Opt. Soc. Am. A, 1993

SUMMARY OF THE INVENTION

Most of the above-described techniques need subject to convey his or her perception based on awareness, i.e., whether he or she could have visually recognized the object, to a doctor etc., either orally or by button pressing. The above-described conventional techniques, however, have disadvantages.

Specifically, information conveyed from the subject or measurement results may include intentional distortion by the subject, namely, a possibility exists that subjects may provide erroneous information. Under the circumstances where erroneous information can be provided intentionally or under wrong impression from the subject, sufficiently objective data may not be obtained.

Also, according to the conventional techniques, subjects are requested to understand instructions from test organizer and, afterwards, report the test results orally and by moving their hands voluntarily in accordance with the instructions. Those procedures, however, may not be applied to persons who are unable to understand the instructions or respond to the questions, such as infant and physically or mentally challenged people.

Accordingly, the existing evaluating techniques may not be applicable to a longitudinal survey of visual function for humans in the earliest stages of development. Of course, the subjects are limited to human beings. The above-described non-patent literature 1 fails to disclose any non-invasive, objective, and quantitative method for identifying a visual filter representing a basic property of the visual function by using a reflective eye movement.

MEANS FOR SOLVING THE PROBLEMS

To solve the problems, the present invention is to provide an embodiment for identifying a visual filter, which comprises the steps of:

(a) sequentially presenting, on a monitor disposed in front of a subject, an initial image having a constant luminance in an entirety of the initial image, a first stimulus pattern having a mean luminance that is the same as that of the initial image, and a second stimulus pattern that induces an apparent motion in conjunction with the first stimulus pattern;

(b) measuring an eye movement within a period while the second stimulus pattern is being presented and storing the measured eye movement in association with a presentation time length of the first stimulus pattern;

(c) repeating steps (a) and (b) as a trial to have a series of trials, each of which has varied settings of the presentation time length for the first stimulus pattern;

(d) calculating a change in eye position according to data of the measured eye movement for each of the trials in step (c);

(e) inputting the first and second stimulus patterns into a motion energy model of eye movement to calculate a simulation result;

(f) optimizing parameters of the motion energy model so that a difference between a measurement waveform identified by the change in eye position acquired in step (d) and by the presentation time length associated with the change in eye position and the simulation result acquired in step (e) is minimized and thereby calculating a temporal filter that is specific to the subject.

ADVANTAGEOUS EFFECTS OF THE INVENTION

According to the invention, a visual filter representing basic characteristics of visual function is non-invasively, objectively, and quantitatively identified by using the eye movement response.

BRIEF DESCRIPTION OF THE DRAWINGS

Figure 1 is a diagram schematically illustrating a visual filter identifying system used in the experiment.

Figure 2 is a diagram illustrating an arrangement of images forming one trial in MOD test.

Figure 3 is a diagram illustrating a measurement period that is used for identifying a temporal filter in MOD test.

Figure 4 is a diagram illustrating data of change in eye velocity measured in the trials of MOD test.

Figure 5 is a diagram in which the data of change in eye velocity is arranged in line according to the presentation time length of the first stimulus pattern.

Figure 6 is a diagram illustrating a response characteristic that is measured by MOD test.

Figure 7 is a diagram illustrating an optimization of an output (simulation result) of a motion energy model.

Figure 8 is a diagram illustrating an example of an identified temporal filter.

Figure 9 is a diagram illustrating a frequency characteristic of visual filter.

Figure 10 is a diagram illustrating an arrangement of images forming one trial in ISI test.

Figure 11 is a diagram illustrating a measurement period that is used for identifying the temporal filter in ISI test.

Figure 12 is a diagram illustrating data of change in eye velocity measured during multiple trials in ISI test.

Figure 13 is a diagram in which the data of change in eye velocity in Figure 12 is arranged according to the presentation time length of the second gray image.

Figure 14 is a diagram illustrating a response characteristic that is measured in a combination of MOD and ISI tests.

EMBODIMENTS OF THE INVENTION

With reference to the accompanying drawings, embodiments according to the invention will be described below. The present invention should not be limited to the specific embodiments described below and they can be modified in various ways without departing from the gist of the invention.

1. First Embodiment

1-1: Structure of system for identifying visual filter

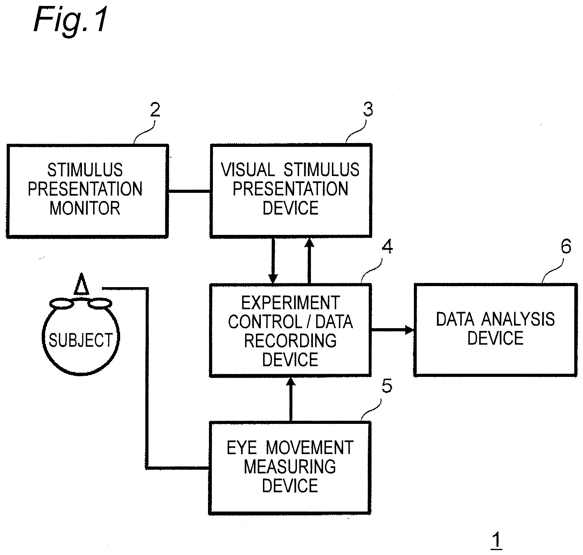

Figure 1 illustrates a schematic structure of a visual filter identifying system generally indicated by reference numeral 1, which was employed in the experiments conducted by the inventors. The visual filter identifying system 1 has a stimulus presentation monitor 2, a visual stimulus presentation unit 3, an experiment control and data recording unit 4, an eye movement measuring unit 5, and a data analysis unit 6. Among others, each of the visual stimulus presentation unit 3, the experiment control and data recording unit 4, and the data analysis unit 6 may be made of a general-purpose computer. In this instance, functions to be performed in each unit may be implemented through an execution of programs stored in the computer.

Each computer has an input/output unit for exchanging data with external units, a memory unit for memorizing data, a control device for controlling executions of programs and conditions of various units associated therewith, and a processor for executing data calculation or data processing. In the following descriptions, each of the visual stimulus presentation unit 3, the experiment control and data recording unit 4, and the data analysis unit 6 is made of an independent computer. Alternatively, whole or parts of the functions of those units may be performed by a single computer.

The stimulus presentation monitor 2, which presents a variety of images to be used in identifying a temporal filter, is disposed in front of subject such as human being or animal to be tested. Example of the stimulus presentation monitor 2 may be a flat panel display such as Cathode Lay Tube, Liquid Crystal Display, or Organic Electro-Luminescence Display or a projector. In the experiments, 19-inch CRT monitor with a size of 360 mm by 270 mm, resolution of 1280 by 1024 pixels, and reflex rate of 100Hz was used.

In the experiments, for the purpose of obtaining precise measurement data, a head of the subject is immovably supported during measurement. Preferably, the head of human subject is fixed by forcing his or her chin against an appropriate support (chin rest) or by having human subject bite a teeth-shaped object called bite block. In the embodiment, a distance between the subject and the monitor display was set to be 63.4 cm, which is a mere example and may be changed as necessary.

The visual stimulus presentation unit 3 is designed to generate certain images to be displayed on the stimulus presentation monitor 2, including a first stimulus pattern and a second stimulus pattern which will be described below. In the embodiment, a commercially available software "MAT LAB®" and a free software "Psychtoolbox" developed in the field of psychology were used for generating images.

The experiment control and data recording unit 4 cooperates with the visual stimulus presentation unit 3 to control the output images. In the embodiment, a software "Real-time Experimental System (REX)" developed by National Institute of Health (NIH) was used. REX may be replaced by commercially available software under the tradename "LabView". The experiment control and data recording unit 4 includes hardware components, such as at least A/D converter and User Database Protocol (UDP) communication device.

Eye position of the subject is also recorded by REX. According to the embodiment, voltages indicating respective eye positions are transformed by the A/D converter into a 12-bit digital data. The transformed data is collected every one millisecond and recorded in, for example, hard disk. The experiment control and data recording unit 4 instructs the visual stimulus presentation unit 3 to display an image of circular fixation target with a diameter (viewing angle) of 0.5 degrees at upper, lower, left, and right positions 5.0 degrees away from the center of the display, and also stores the output voltage measured during the presentation of the fixation target to which the subject directs his/her line of sight by the eye movement measuring unit 5 as respective references in the hard disk, for example. By using the reference voltages obtained during the presentation of the fixation targets, voltages to be measured during the subsequent presentation of apparent motion stimuli can be used for the calculation of rotational angle of eye.

The eye movement measuring unit 5, which is designed to measure the eye movement generated by the presentation of apparent motion inducing images, continues to measure the eye measurement while the subject is watching the monitor. Alternatively, the eye movement may be measured only in a limited period of time necessary for data analysis which will be described below, or in a period of time during which images including gray image is displayed on the stimulus presentation monitor 2.

The eye movement can be measured through various methods known in the art, such as method using first and fourth Purkinje's images, search coil method, and Limbus tracking method. Another method may be used instead, in which the eye movement of the subject is captured by a video camera and then extracted from the captured image. In the embodiment, the eye position (gaze direction) of the subject is measured by an eye movement tracking system commercially available from Fourward Technology under the tradename "Dual-Purkinje Image Eye Tracker" which uses the first and fourth Purkinje's images. The eye position is obtained in the form of voltage signal transmitted from the eye movement tracking system.

The data analysis unit 6 is designed to process certain calculations for analyzing data obtained during the presentation of images prepared for inducing apparent motion and thereby identifying the visual filter (including temporal filter) inherent to the subject. The temporal filter may be identified by optimizing parameters of the motion energy model so that simulation result calculated from the motion energy model of the eye movement response becomes substantially identical to the measurement result, which will be described in detail below. In this application, the "energy model of eye movement response" is used to indicate any energy-based motion detector model proposed for describing the eye movement response. In this embodiment, a model proposed in 1985 by Adelson and Bergen for describing a subjective perception of the movement is used for the energy model of eye movement response. Another model such as Elaborated Reichardt model may be used instead.

1.2 Motion Onset Delays (MOD) Test

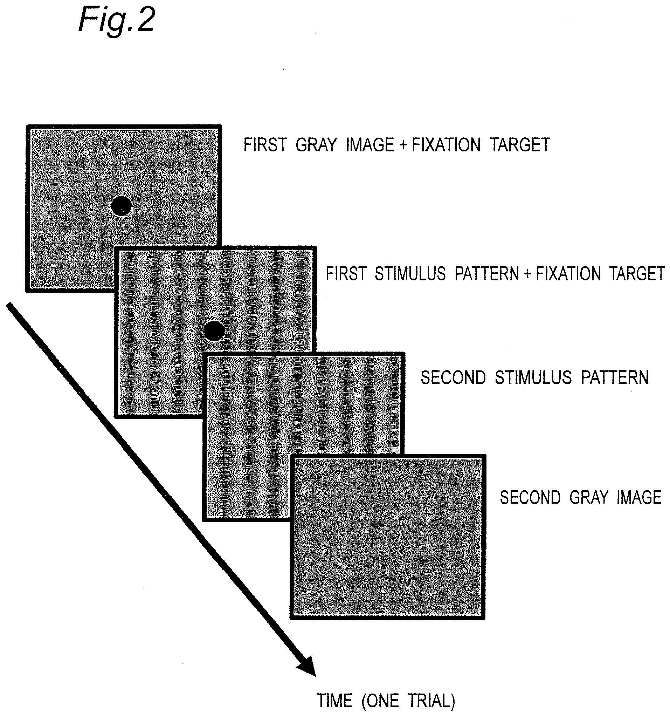

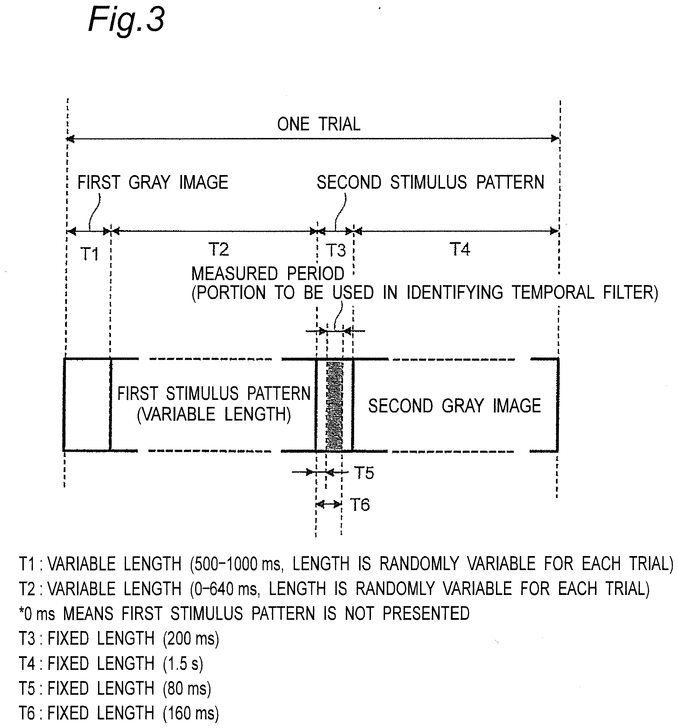

In the embodiment, so-called Motion Onset Delays (MOD) Test is used for measuring a reflexively induced eye movement response, in which an image presentation unit or trial is repeated a plurality of times to collect measurement data necessary for identifying a temporal filter of the subject. Figures 2 and 3 indicate a series of images defining an image presentation unit or trial in MOD test. In each trial, the series of images for each trial are presented by the visual stimulus presentation unit 3 under the control of the experiment control and data recording unit 4.

Each trial has successive four time lengths for displaying, on the monitor, (1) a combination of a fixation target and a first gray image, (2) a combination of the fixation target and a first stimulus pattern, (3) a second stimulus pattern, and (4) a second gray image, in this order. In the embodiment, each of the first and second gray images has in its entirety a mean luminance of, for example, 5cd/m2. The luminance of the first gray image may be different from that of the second gray image. Preferably, the luminance of the first gray image is the same as the mean luminance of the first and second stimulus patterns.

Each trial begins with a presentation of the first gray image with the circular fixation target having an angle diameter of 0.5 degrees and positioned at the center of the first gray image. In this embodiment, a time length for presenting the first gray image is randomly varied between 500 milliseconds to 1,000 milliseconds. The time length for presenting the first gray image may be fixed between 500 milliseconds to 1,000 milliseconds. After elapse of the time length, the fixation target and the first stimulus pattern are presented instead on the display.

In the embodiment, a vertical sinusoidal grating image is used for the first stimulus pattern of which luminance varies sinusoidally in the horizontal direction with a spatial frequency of 0.25 cycles/degree, a Michelson contrast of 32%, and a mean luminance of 5.0 cd/m2. The luminance of the first stimulus pattern may be expressed with 2048-tone gray levels. The presentation time length for the first stimulus pattern is varied randomly so that it will be different at each trial. This is for preventing the subject from predicting the presentation time length. As long as the test result is assured not to be affected by the prediction, the presentation time length is not necessarily varied randomly. In this case, the presentation time length may be modified at each trial according to a predetermined pattern.

In the embodiment, different time lengths 0, 10, 20, 30, 40, 60, 80, 160, 320, and 640 milliseconds are randomly assigned to the first stimulus patterns of respective trials in a block. The time length "0" millisecond means that no first stimulus pattern is presented.

If different 10 types of time lengths are provided, they are randomly assigned to respective 10 trials so that the same presentation time length will not be used during the 10 trials. It is not desirable that the presentation time length monotonically increases or monotonically decreases during the 10 trials.

The first stimulus pattern may be determined to induce apparent motion when it is combined with the second stimulus pattern. The first stimulus pattern is not limited to the vertical sinusoidal grating image described above, and it may be a horizontal sinusoidal grating image of which luminance varies sinusoidally in the horizontal direction, an oblique sinusoidal grating image of which luminance varies sinusoidally in the oblique direction, or a plaid pattern of which luminance varies in a plaid fashion. The luminance needs not to be varied sinusoidally, and it may be changed alternately between two levels in the form of square wave.

After elapse of the time length for the presentation of the first stimulus pattern, the fixation target disappears from the screen of the monitor, and at the same time the presentation of the second stimulus pattern begins. The above mentioned Motion Onset Delay (MOD) is a period of time that starts from the beginning of the presentation of the first stimulus pattern to the beginning of the presentation of the second stimulus pattern (i.e., a presentation time of the first stimulus pattern).

In the embodiment, the second stimulus pattern is similar to the first stimulus pattern, i.e., vertical sinusoidal grating image, except that a phase of the second stimulus pattern is shifted leftward or rightward by 90 degrees (i.e., 1/4 wavelength) with respect to the first stimulus pattern. The phase shift ($\theta$) may be selected to lie in the range $0<\theta<180°$. In the embodiment, the second stimulus pattern is presented for 200 milliseconds, for example.

A reflexive eye movement of the subject, which occurs by changing images on the display monitor from the first stimulus pattern to the second stimulus pattern, is measured to obtain associated data in a period of time that begins after 50 milliseconds and ends after 200 milliseconds (preferably after 80 milliseconds and ends after 160 milliseconds) from starting the presentation of the second stimulus pattern, as shown in Figure 3, because the reflexive eye movement appears after the change of stimulus patterns. This period of time for obtaining data described above is a mere example and it can be appropriately determined in consideration of contrast and/or spatial frequency of the stimulus patterns.

Preferably, in order to remove trial data which is considered to include saccadic eye movement described below (i.e., gaze direction shifts due to rapid eye movements) in the process of data analysis, the recording of the eye movement is started a certain period of time (for example, 50 milliseconds) before initiating the presentation of the second stimulus pattern.

After elapse of the presentation of the second stimulus pattern, the second gray image is displayed on the monitor screen. No fixation target is presented with the second gray image. In the embodiment, a time length for presenting the second gray image is about 1.5 milliseconds. After elapse of this time length, a next trial will be started.

In the embodiment, a block consists of two sets each having 10 trials. In each set, different time lengths are assigned to 10 trials. The first and second stimulus patterns are moved in the left or right direction in one set and in the opposite direction in the other set. In other embodiments, the stimulus patterns may be moved in the same direction. An increased number of presentation time lengths or motion direction patterns may be applied. The stimulus patterns may be moved not only in the horizontal directions but also in other directions. Preferably, the test includes at least two trials in which the first stimulus pattern has the same time length and is moved in the same direction, which ensures that noises are reduced by averaging measurement data obtained through those trials performed under the same condition. In an embodiment, the test includes 30 blocks in which 600 trials (20 trials for each of 30 blocks) are conducted.

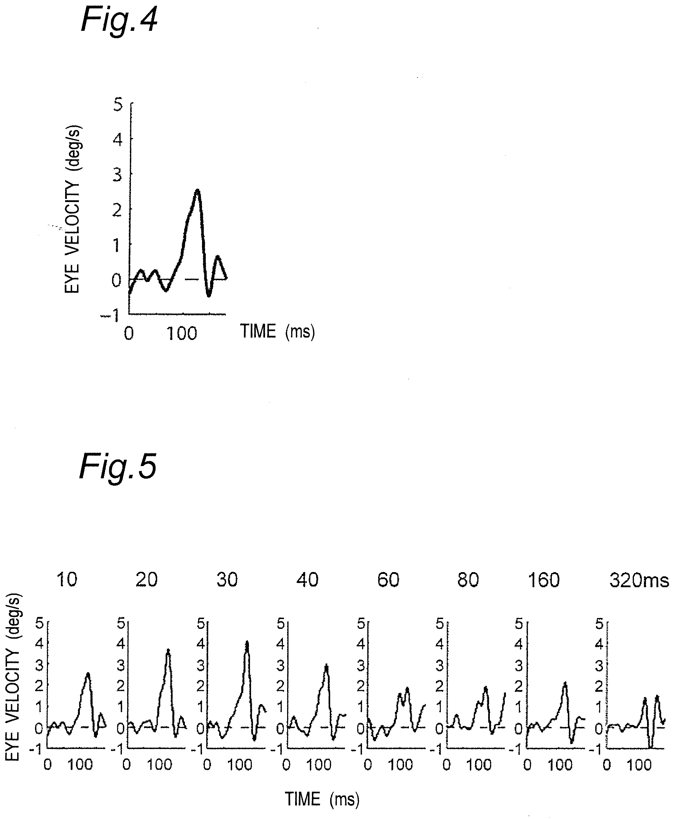

Figure 4 illustrates an eye velocity profile which is obtained by processing data acquired from the measurements of eye positions conducted under condition that the first stimulus pattern was presented for 10 milliseconds. Specifically, the eye velocity profile is obtained by subtracting average eye velocity data acquired in the trials in which the stimulus pattern was moved in the left direction from the average eye velocity data acquired in the test in which the stimulus pattern was moved in the right direction. The horizontal axis represents a time (millisecond) elapsed from staring the presentation of the second stimulus pattern in the measurement period of time indicated in Figure 3. The vertical axis represents an eye velocity.

The differential processing for obtaining the eye velocity from the eye position is performed in the data analysis unit 6. The experiment control and data recoding unit 4 associates the time lengths of the first stimulus pattern and the directions of the apparent motion stimulus with the measurement data in each trial and stores the associated data into a hard disk, for example. Then, the measurement data illustrated in Figure 5 is recorded in the hard disk at the completion of the all trials. It should be noted that Figure 4 simply describes how the eyes move. In the practical data analysis, data of eye position change for each of respective presentation time lengths is calculated from the measurement data of eye position. In this instance, the eye velocity data obtained through trials are not necessarily averaged.

Figure 5 illustrates the measurement data for respective presentation time lengths of the first stimulus pattern (corresponding to eye velocity profile shown in Figure 4), in which the measurement data is relocated according to the time length of the first stimulus pattern. A value obtained by an integration of data from 80 milliseconds to 160 milliseconds indicates the movement or displacement of eye for the time length of first stimulus pattern, which is the basic data for identifying a temporal filter that is specific to the subject. Figure 5 is also provided to simply describe how the eyes move. In the practical data analysis, data of eye position change for each of respective presentation time lengths is calculated from the measurement data of eye position without obtaining average eye velocity data.

1-3 Data Analysis

Hereinafter, discussions will be made to a data analysis performed in the data analysis unit 6. The data analysis unit 6 obtains the measurement data from the experiment control and data recording unit 4 and analyzes the data by using MATLAB®. In this process, the data of eye position change for respective time lengths of the first stimulus patterns is analyzed using the motion energy model to identify the temporal filter. The analysis is performed in the following steps:

(1) The data analysis unit 6 uses a digital low-pass filter (for example, 4-pole Butterworth Filter, -3dB at 25Hz) to remove high-frequency noises from the eye position data.

(2) The data analysis unit 6 differentiates the eye position data free from noises with respect to time to obtain the eye velocity data.

(3) The data analysis unit 6 differentiates the eye velocity data to obtain the eye acceleration data.

(4) The data analysis unit 6 removes data of trials in which saccadic eye movement (i.e., gaze direction shifts due to rapid eye movements) occurred in a period of time that begins 50 milliseconds before the presentation of the second stimulus pattern (in a time period in which the first stimulus pattern is being presented) and ends 200 milliseconds after the presentation of the second stimulus pattern. In this embodiment, the saccadic eye movement is defined as the eye movement with a velocity of more than 30 degrees/second or with an acceleration of more than 1,000 degrees/square seconds.

The data analysis unit 6 uses only the measurement data of the trials free from saccadic eye movement to calculate the change in eye position from the measurement data obtained in a period of time that begins 80 milliseconds and ends 160 milliseconds from the starting of the presentation of the second stimulus pattern. In the embodiment, the change in eye position is calculated by subtracting data of eye position at 160 milliseconds from that at 80 milliseconds, from the starting of the presentation of the second stimulus pattern. Alternatively, the eye position change may be calculated by integrating eye velocity during the time period of 80-160 milliseconds.

(6) The data analysis unit 6 averages the measurement data acquired in the trials performed under the same stimulating condition in order to obtain eye movement response for each presentation time length.

(7) In order to improve the signal-noise (S/N) ratio, the data analysis unit 6 calculates a difference between the average of the eye movement response obtained in a condition where the second stimulus pattern is moved in the right direction with respect to the first stimulus pattern and the average of the eye movement response obtained in a condition where the second stimulus pattern is moved in the left direction with respect to the first stimulus pattern. The eye movement responses in opposite directions have different, positive and negative values, which results in that the subtraction of the eye movement responses in opposite directions substantially doubles the mean value of those two eye movement responses.

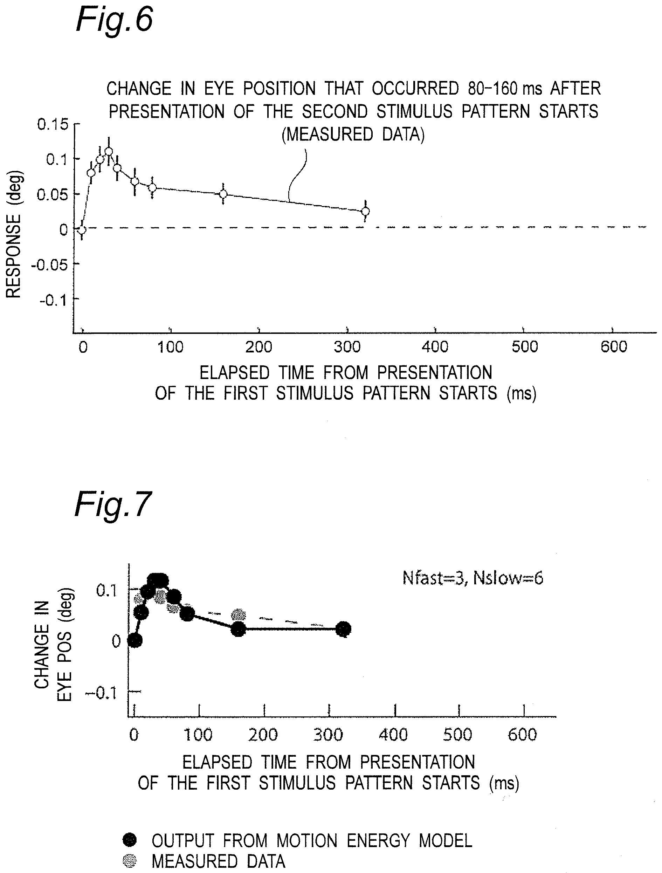

Through the calculations described above, the data analysis unit 6 obtains a response characteristic (measurement data) of the subject against the stimulus condition. Figure 6 illustrates a diagram showing a measured response characteristic. The horizontal axis represents the time length for presenting the first stimulus patterns. The vertical axis represents a response, namely, change in eye position in the time range of 80-160 milliseconds after starting of the second stimulus pattern.

Then, the data analysis unit 6 inputs images obtained under the same stimulus condition as those at the measurement into a motion energy model (proposed by Adelson & Bergen in 1985) with a temporal filter defined by the following equation to calculate a simulation result for each stimulus condition:

[Equation 1]

$$y(t) = (kt)^N e^{(-kt)} \left( \frac{1}{N!} - b \frac{(kt)^2}{(N+2)!} \right)$$

where k, b, and N are the time constant, the strength of the negative lobe, and the order, respectively. The motion energy model includes two temporal filters-Fast filter and Slow filter. The Fast and Slow filters have same k and b, but different orders. In this embodiment, the orders of Fast and Slow temporal filters are assumed to be $N_{fast}$ and $N_{slow}$ ($>N_{fast}$), respectively. For the calculation of the first simulation, initial values prepared for respective parameters are used.

The data analysis unit 6 repeatedly inputs data of trials each having first and second stimulus patterns with different time lengths for the first stimulus patterns into the above motion energy model to calculate simulation results for respective inputs. The output of the motion energy model is a difference of energy between the leftward and rightward motions (hereinafter referred to as "output of motion energy model"), which is provided as a function of time.

In this embodiment, a mean value of the motion energy model over a period of time from 0 to 80 milliseconds is used as a simulation result. This period of time is not restrictive. Instead of the mean value, an integrated value of the motion energy model over the period may be used as a simulation result.

(9) The data analysis unit 6 optimizes parameters (*k, b*) determining the property of the temporal filter in the motion energy model and a scaling constant (C) for adjusting the output of the motion energy model. In this embodiment, the optimization is performed with the orders of Fast and Slow temporal filters $N_{fast}$ and $N_{slow}$ fixed at "3" and "6", respectively. The temporal filters $N_{fast}$ and $N_{slow}$ may also be optimized. As such, the parameters of the motion energy model are optimized so that a difference between the simulation result and the mean value of the eye movement responses for respective presentation time lengths is minimized. Instead of adjusting the temporal waveform of eye movement, the optimization is performed so that the model output matches with an average of amplitudes of eye movement response that depends on the presentation time length for the first stimulus pattern.

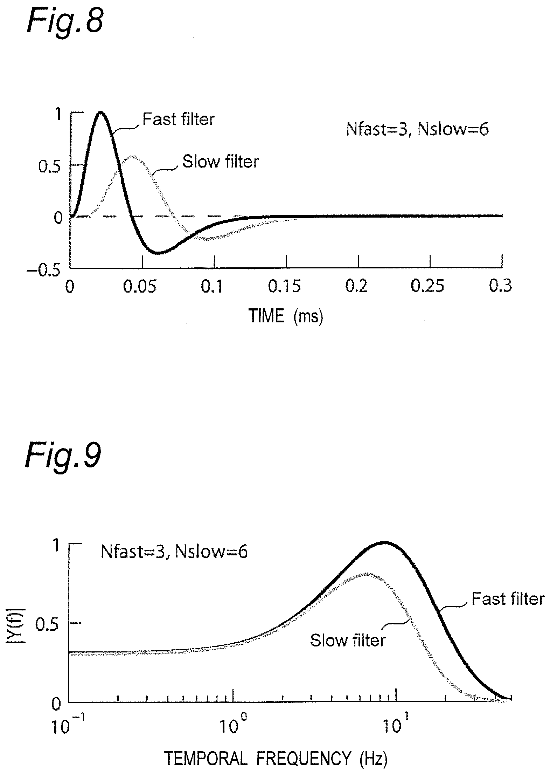

An existing algorithm using nonlinear optimization method such as nonlinear least-square method may be applied to the optimization. Figure 7 visualizes the optimization process. Once the parameters are determined so that the difference between the simulation result and the response property (measurement data) quantified for the respective stimulus conditions is minimized, the temporal filter defining the temporal resolution in the visual system of the subject is quantitatively identified. Figure 8 illustrates an example of the temporal filter identified, which is well supported by the measurement data.

The data analysis unit 6 applies Fourier transformation on the identified temporal filter to obtain frequency characteristics of the temporal filter. Figure 9 shows an example of the frequency characteristics of the temporal filter so obtained. In this specification, the term "visual filter" is used to include both a filter representing the frequency characteristics and the temporal filter described above. A variety of quantitative information representing the visual properties such as optimal time frequency, passband frequency band, and optimal velocity may be obtained from the visual filter.

1-4 Advantages

As described above, the visual filter identifying system 1 according to the embodiment described above, as it uses the eye movement response in identifying the visual filter, allows the temporal filter representing the temporal resolution of the visual system to be non-invasively, objectively, and quantitatively identified. Then, there is no need for the subject to understand instructions from test organizer or respond by moving his or her body. Also, there is no room for the subject to introduce his or her intentional distortion and, therefore, a sufficiently objective data can be obtained.

The technology described with reference to the embodiment is to identify the most fundamental function (visual filter) in the visual system and, therefore, may be used as a quantitative diagnosis method for diagnosing normality/abnormality of retina, optic nerve, primary visual cortex, higher visual cortex (areas in occipital and pariental association cortex that are related to motion and spatial vision). The technology may also be applied in developing a test method for evaluating effect on treatment, rehabilitation, or training.

The technology may further be applied in the study of fundamental visual function and apparatus therefor in a variety of diagnoses in the medical or welfare field (department of pediatrics, ophthalmology, neurology, psychiatry, and rehabilitation). The subject may be (1) infants, children, and adults, (2) patients who are unable to make appropriate response to any questions, and (3) animals rather than human beings.

The technology may be applied a plurality of times to the same human subject because it uses reflexive eye movement response, which in turn allows a longitudinal examination for evaluating the development of the subject and/or treatment effect. For example, it may be used in the device or products for evaluating changes in visual function due to development and aging, or disturbance of visual function due to mental illness.

The visual filter, which can be quantified according to the embodiment, is a one factor for determining dynamic vision. Although the conventional test for determining the dynamic vision is to measure an ability of spatial resolution relating to how clearly the subject can see the moving object, the technology described in the embodiment is to measure the temporal resolution for viewing changes of visual image and is directed to recognize the movements of target, namely, it provides an inventive technique for measuring a dynamic vision of subject which has never been recognized up to now and is expected to be applied to a new visual function examination in evaluating the visual capability of athletes and a product development in the industry of education and physical training.

Further, the technology may be installed in an experimental device for test animals. Furthermore, the evaluation method may be applied to animals such as mouse and is expected to be used in scientific researches and new drag development.

2. Second Embodiment

2.1 InterStimulus Interval (ISI) Test

The MOD test described in the previous embodiment may be used in combination with ISI test. The ISI test is to measure an eye movement response which would be induced by presenting a combination of the first and second stimulus patterns for inducing apparent motion in the MOD test and an additional image having a mean luminance of the first and second stimulus patterns, in which the subject perceives a motion in a direction opposite to that the shift of the second image pattern with respect to the first image pattern.

An order of the measurements of MOD and ISI tests is not limited. For example, ISI measurement is performed after MOD measurement; MOD measurement is performed after ISI measurement; MOD and ISI measurements are performed alternately; or MOD and ISI measurements are performed at random. Discussions will be made to ISI test which uses a visual filter identifying system 1 (Figure 1).

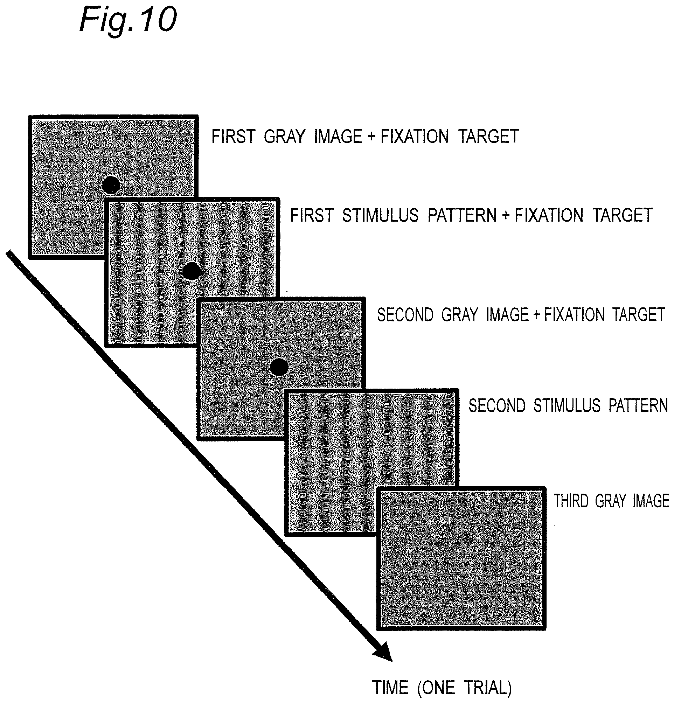

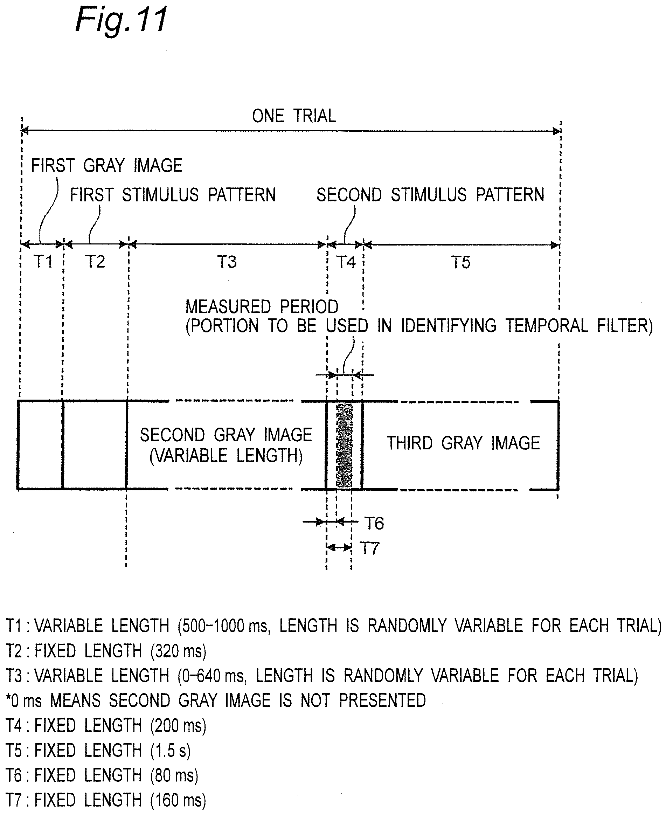

Figures 10 and 11 illustrate a series of images to be displayed in one trial of ISI test. The presentation of the images for one trial (see Figures 10 and 11) is performed by the stimulus presentation unit 3 under the control of the experiment control and data recording unit 4.

As illustrated in Figure 10, each trial has successive five time lengths for displaying, on the monitor, (1) a combination of a fixation target and a first gray image, (2) a combination of the fixation target and a first stimulus pattern, (3) a combination of the fixation target and a second gray image, (4) a second stimulus pattern, and (4) a third gray image, in this order. A substantial difference between the first embodiment and the second embodiment is that, according to the second embodiment, the fixation target and the second gray image are presented between the first and second stimulus patterns.

In this embodiment, each of the first, second, and third gray images has in its entirety a mean luminance of, for example, 5 cd/m2. The luminance of the first and second gray images may be the same as the mean luminance of the first and second stimulus patterns.

As illustrated in Figure 11, the time length for presenting the fixation target and the first gray image may be changed randomly in the range from 500 milliseconds to 1,000 milliseconds so that each trial has a time length that is different from those of other trials. The time length may be fixed in the range from 500 milliseconds to 1,000 milliseconds, for example. After elapse of this time length, the fixation target and the first stimulus pattern is presented on the monitor display. The first stimulus pattern may be the same as that in the first embodiment. A vertical sinusoidal grating image is used for the first stimulus pattern, of which luminance varies sinusoidally in the horizontal direction with a spatial frequency of 0.25 cycles/degree, a Michelson contrast of 32%, and a mean luminance of 5.0 cd/m2. The time length of the first stimulus pattern is fixed to 320 milliseconds.

After elapse of this time length, the fixation target and the second gray image are presented on the monitor display. The time length of the second gray image is varied randomly so that each trial has a time length that is different from other trials in order that the subject is unable to predict the time length of the image.

In the embodiment, different time lengths 0, 10, 20, 30, 40, 60, 80, 160, 320, and 640 milliseconds are randomly assigned to the second gray images of respective trials. The time length "0" millisecond means that no first stimulus pattern is presented.

Specifically, for the first stimulus pattern, 10 time lengths are randomly assigned to respective 10 trials so that the same time length is not provided for plural trials in a block. The time lengths should be selected not to gradually increase or decrease with the number of trials.

After elapse of this time length, the fixation target disappears from the monitor display, and the second stimulus pattern appears instead. The second stimulus pattern is the same as that in the first embodiment. The second stimulus pattern is the same as the first stimulus pattern, except that a phase of the second stimulus pattern is shifted leftward or rightward by 90 degrees (i.e., 1/4 wavelength) with respect to the first stimulus pattern. In this embodiment, the second stimulus pattern is presented for 200 milliseconds, for example. A period of time for measuring the reflectively induced eye movement is the same as that in the first embodiment.

After elapse of the time length of the second stimulus pattern, the third gray image is presented on the monitor display. No fixation target is presented with the third gray image. In this embodiment, a time length for presenting the third gray image is about 1.5 milliseconds. After elapse of this time length, a next trial will be started. The number of the trials is the same as that in the first embodiment.

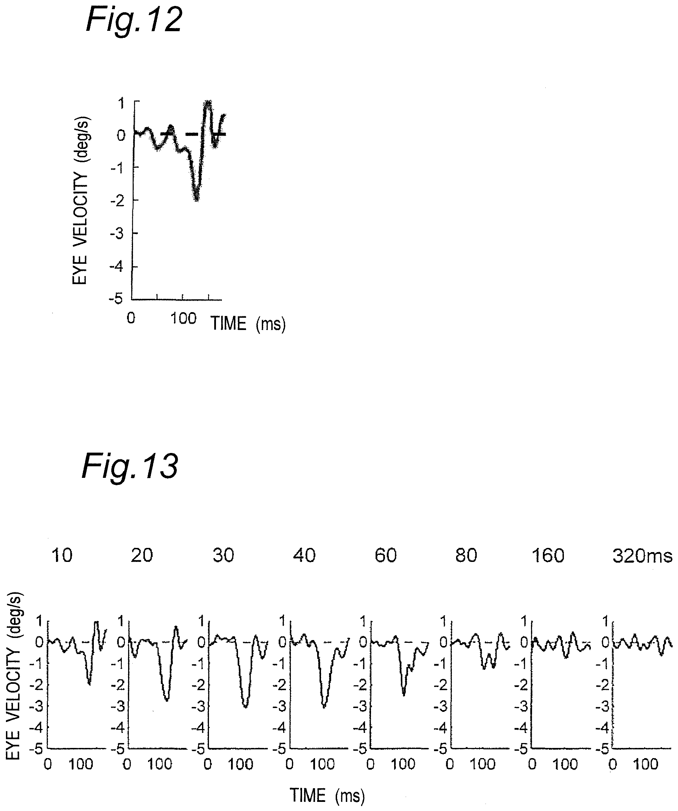

Figure 12 illustrates an eye velocity profile which is obtained by processing data acquired from the measurements conducted under the condition that the second gray image was presented for 10 milliseconds. Specifically, the eye velocity profile is obtained by subtracting average eye velocity data acquired in the trials in which the stimulus pattern was moved in the left direction from the average eye velocity data acquired in the test in which the stimulus pattern was moved in the right direction. The horizontal axis in Figure 12 represents a time (millisecond) elapsed from staring the presentation of the second stimulus pattern. The vertical axis represents an eye velocity. The eye velocity is calculated by the data analyzing unit 6. The experiment control and data recording unit 4 associates time lengths of the second gray image and the direction of the stimulus shift with the measurement data of each trial and stores the same in the hard disk, for example. After the completion of all trials, the measurement data illustrated in Figure 13 is recorded in the hard disk.

Figure 13 illustrates the measurement data for respective presentation time lengths of the second gray image (corresponding to eye velocity profile shown in Figure 12), in which the measurement data is relocated according to the time length of the second gray image. The change in eye position during the 80-160 milliseconds, corresponding to a value to be obtained by an integration of data for that period of time, is the basic data for identifying a temporal filter unique to the subject. A data analysis for ISI test is the same as that for MOD test and, therefore, no description will be made thereto.

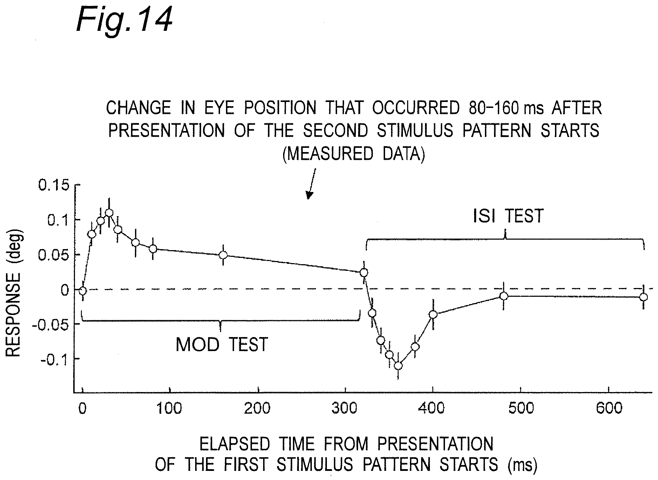

Figure 14 illustrates a diagram showing the measurement data of the response characteristic obtained by MOD test and the measurement data of the response characteristic obtained by ISI test, arranged on the same time axis. In the drawing, the measurement data of ISI test is indicated following the MOD test because the time length of the first stimulus pattern is 320 milliseconds. In this drawing, the maximum time length of the first stimulus pattern of MOD test is 320 milliseconds.

2-2 Effects

As described above, a visual filter including not only the eye movement but also subjective visual perception is non-invasively, objectively, and quantitatively identified by using a single visual filer identifying system 1 and performing MOD and ISI tests while changing images displayed on the monitor display.

PARTS LIST

1: visual filter identifying system
2: stimulus presentation monitor
3: visual stimulus presentation unit
4: experiment control and data recording unit
5: eye movement measurement unit
6: data analysis unit

**Claims**

1.  A method for identifying a visual filter, comprising the steps of:

    (a) sequentially presenting, on a monitor disposed in front of a subject, an initial image having a constant luminance in an entirety of the initial image, a first stimulus pattern having a mean luminance that is the same as that of the initial image, and a second stimulus pattern that induces an apparent motion in conjunction with the first stimulus pattern;
    (b) measuring an eye movement within a period while the second stimulus pattern is being presented and storing the measured eye movement in association with a presentation time length of the first stimulus pattern;
    (c) repeating steps (a) and (b) as a trial to have a series of trials, each of which has varied settings of the presentation time length for the first stimulus pattern;
    (d) calculating a change in eye position according to data of the measured eye movement for each of the trials in step (c);
    (e) inputting the first and second stimulus patterns into a motion energy model of eye movement to calculate a simulation result;
    (f) optimizing parameters of the motion energy model so that a difference between a measurement waveform identified by the change in eye position acquired in step (d) and by the presentation time length associated with the change in eye position and the simulation result acquired in step (e) is minimized and thereby calculating a temporal filter that is specific to the subject.

2.  The method of claim 1,
    wherein the change in eye position calculated in step (d) has representative values; and the representative values are generated respectively, as average values of the measured eye movements for trials that share an identical presentation time length.

3.  The method of claim 1,
    wherein each of the steps (a) and (b) comprises a first trial and a second trial, wherein the first trial and the second trial are performed for each of the presentation time lengths of first stimulus pattern, wherein in the first trial the second stimulus pattern is presented to induce the apparent motion in a first direction and in the second trial the second stimulus pattern is presented to induce the apparent motion in a second direction opposite to the first direction, wherein in step (d) a difference between eye movement responses in the first trial and the second trial is calculated for each of the presentation time lengths, and the calculated difference is used as the representative value of the change in eye position.

4.  The method of claim 1, further comprising:

    (g) performing Fourier analysis on the temporal filter to identify a characteristic of frequency distribution.

5.  The method of claim 1,

wherein the period in step (b) is a period of 50 milliseconds to 200 milliseconds after the presentation of the second stimulus pattern starts.

6. The method of claim 1,
wherein the first stimulus pattern and the second stimulus pattern have a spatial frequency, and
wherein the second stimulus pattern is phase-shifted by an angle ($\theta$) lying between 0 to 180 degrees with respect to the first stimulus pattern.

7. The method of claim 1,
wherein a fixation target is indicated with the initial image and with the first stimulus pattern.

8. A system for identifying a visual filter comprising:

a visual stimulus presentation unit that presents, on a monitor disposed in front of a subject, an initial image having a constant luminance in an entirety of the initial image, a first stimulus pattern having a mean luminance that is the same as that of the initial image, and a second stimulus pattern that induces an apparent motion in conjunction with the first stimulus pattern, wherein the visual stimulus presentation unit repeats a plurality of times a trial for presenting the initial image, the first stimulus pattern, and the second stimulus pattern while assigning each trial with a presentation time length of the first stimulus pattern that is different from those of other trials;
a data recording unit that records data of eye movement obtained in a period during a presentation of the second stimulus pattern in association with the presentation time length of the fist stimulus pattern; and

a data analyzing unit comprising

a first calculator that calculates a change in eye position according to the data of the eye movement for each of the presentation time length;
a second calculator that inputs the first and second stimulus patterns into a motion energy model of eye movement response to calculate a simulation result; and
a third calculator that optimizes parameters of the motion energy model so that a difference between a measurement waveform identified by the presentation time length and by the change in eye position corresponding to the presentation time length and the simulation result is minimized and thereby calculating a temporal filter that is specific to the subject.

## Fig.1

```
     2                              3
STIMULUS              VISUAL STIMULUS
PRESENTATION          PRESENTATION
MONITOR               DEVICE
                                        4                      6
           ▲                      EXPERIMENT          DATA ANALYSIS
        SUBJECT                   CONTROL / DATA       DEVICE
                                  RECORDING
                                  DEVICE
                                              5
                                  EYE MOVEMENT
                                  MEASURING
                                  DEVICE
```

1

## Fig.2

FIRST GRAY IMAGE + FIXATION TARGET

FIRST STIMULUS PATTERN + FIXATION TARGET

SECOND STIMULUS PATTERN

SECOND GRAY IMAGE

TIME (ONE TRIAL)

# Fig.3

T1 : VARIABLE LENGTH (500-1000 ms, LENGTH IS RANDOMLY VARIABLE FOR EACH TRIAL)
T2 : VARIABLE LENGTH (0-640 ms, LENGTH IS RANDOMLY VARIABLE FOR EACH TRIAL)
*0 ms MEANS FIRST STIMULUS PATTERN IS NOT PRESENTED
T3 : FIXED LENGTH (200 ms)
T4 : FIXED LENGTH (1.5 s)
T5 : FIXED LENGTH (80 ms)
T6 : FIXED LENGTH (160 ms)

*Fig.4*

*Fig.5*

TIME (ms)

*Fig.6*

CHANGE IN EYE POSITION THAT OCCURRED 80–160 ms AFTER
PRESENTATION OF THE SECOND STIMULUS PATTERN STARTS
(MEASURED DATA)

ELAPSED TIME FROM PRESENTATION
OF THE FIRST STIMULUS PATTERN STARTS (ms)

*Fig.7*

Nfast=3, Nslow=6

ELAPSED TIME FROM PRESENTATION
OF THE FIRST STIMULUS PATTERN STARTS (ms)

● OUTPUT FROM MOTION ENERGY MODEL
◦ MEASURED DATA

16

*Fig.8*

*Fig.9*

# Fig.10

FIRST GRAY IMAGE + FIXATION TARGET

FIRST STIMULUS PATTERN + FIXATION TARGET

SECOND GRAY IMAGE + FIXATION TARGET

SECOND STIMULUS PATTERN

THIRD GRAY IMAGE

TIME (ONE TRIAL)

## Fig.11

T1 : VARIABLE LENGTH (500-1000 ms, LENGTH IS RANDOMLY VARIABLE FOR EACH TRIAL)
T2 : FIXED LENGTH (320 ms)
T3 : VARIABLE LENGTH (0-640 ms, LENGTH IS RANDOMLY VARIABLE FOR EACH TRIAL)
*0 ms MEANS SECOND GRAY IMAGE IS NOT PRESENTED
T4 : FIXED LENGTH (200 ms)
T5 : FIXED LENGTH (1.5 s)
T6 : FIXED LENGTH (80 ms)
T7 : FIXED LENGTH (160 ms)

## Fig.12

## Fig.13

Fig.14

CHANGE IN EYE POSITION THAT OCCURRED 80-160 ms AFTER
PRESENTATION OF THE SECOND STIMULUS PATTERN STARTS
(MEASURED DATA)

ELAPSED TIME FROM PRESENTATION
OF THE FIRST STIMULUS PATTERN STARTS (ms)

**INTERNATIONAL SEARCH REPORT**

| International application No. |
| --- |
| PCT/JP2017/012067 |

A.  CLASSIFICATION OF SUBJECT MATTER
*A61B10/00(2006.01)i, A61B3/113(2006.01)i*

According to International Patent Classification (IPC) or to both national classification and IPC

B.  FIELDS SEARCHED

Minimum documentation searched (classification system followed by classification symbols)
A61B10/00, A61B3/113

Documentation searched other than minimum documentation to the extent that such documents are included in the fields searched
Jitsuyo Shinan Koho          1922–1996     Jitsuyo Shinan Toroku Koho     1996–2017
Kokai Jitsuyo Shinan Koho    1971–2017     Toroku Jitsuyo Shinan Koho     1994–2017

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | JP 2014-21673 A  (Toshiba Corp.), 03 February 2014 (03.02.2014), claims 1 to 6; paragraphs [0054], [0055] & US 2014/0022279 A1 paragraphs [0057], [0058]; claims 1 to 6 | 1-8 |
| A | JP 2015-72709 A  (Toshiba Corp.), 16 April 2015 (16.04.2015), claims 1 to 5; paragraphs [0054], [0055] (Family: none) | 1-8 |
| A | JP 2014-236944 A  (Issix World Inc.), 18 December 2014 (18.12.2014), claims 1, 4 (Family: none) | 1-8 |

| ☐ Further documents are listed in the continuation of Box C. | ☐ See patent family annex. |
| --- | --- |

\*      Special categories of cited documents:
"A"    document defining the general state of the art which is not considered    to be of particular relevance
"E"    earlier application or patent but published on or after the international filing date
"L"    document which may throw doubts on priority claim(s) or which is cited to establish the publication date of another citation or other special reason (as specified)
"O"    document referring to an oral disclosure, use, exhibition or other means
"P"    document published prior to the international filing date but later than the priority date claimed

"T"    later document published after the international filing date or priority date and not in conflict with the application but cited to understand the principle or theory underlying the invention
"X"    document of particular relevance; the claimed invention cannot be considered novel or cannot be considered to involve an inventive step when the document is taken alone
"Y"    document of particular relevance; the claimed invention cannot be considered to involve an inventive step when the document is combined with one or more other such documents, such combination being obvious to a person skilled in the art
"&"    document member of the same patent family

| Date of the actual completion of the international search         26 April 2017 (26.04.17) | Date of mailing of the international search report         16 May 2017 (16.05.17) |
| --- | --- |
| Name and mailing address of the ISA/         Japan Patent Office         3-4-3,Kasumigaseki,Chiyoda-ku,         Tokyo 100-8915,Japan | Authorized officer           Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BURR ; MORRONE.** *J. Opt. Soc. Am. A,* 1993
- Real-time Experimental System (REX). National Institute of Health (NIH)